(19) Europäisches Patentamt
European Patent Office
Office européen des brevets

(11) Veröffentlichungsnummer: **0 003 555**
**B1**

(12) # EUROPÄISCHE PATENTSCHRIFT

(21) Anmeldenummer: **79100281.9**

(22) Anmeldetag: **31.01.79**

(51) Int. Cl.[3]: **C 07 C 17/10,**
C 07 C 139/02,
C 07 C 143/76,
B 01 J 19/08

(54) **Verfahren zur Herstellung von durch Chloratome und/oder Sulfochloridgruppen substituierten Alkanen**

(30) Priorität: **09.02.78 DE 2805441**

(43) Veröffentlichungstag der Anmeldung:
**22.08.79 Patentblatt 79/17**

(45) Bekanntmachung des Hinweises auf die Patenterteilung:
**10.12.80 Patentblatt 80/25**

(84) Benannte Vertragsstaaten:
**BE CH DE FR GB NL**

(56) Entgegenhaltungen:
**DE - A - 2 217 530**
**DE - A - 2 459 159**
**GB - A - 1 442 122**
**US - A - 2 333 788**
**US - A - 2 335 259**
**US - A - 3 296 108**

(73) Patentinhaber: **BASF Aktiengesellschaft**
**Carl-Bosch-Strasse 38**
**D - 6700 Ludwigshafen (DE)**

(72) Erfinder: **Schlecht, Helmut, Dr.**
**Schuckertstrasse 1**
**D - 6700 Ludwigshafen (DE)**
**Weberndoerfer, Volkmar, Dr. Chem**
**Feldbergstrasse 48**
**D - 6800 Mannheim 1 (DE)**
**Widder, Rudi, Dr. Chem**
**In der Taesch 7**
**D - 6909 Leimen (DE)**

**EP 0 003 555 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von durch Chloratome und/oder Sulfochloridgruppen substituierten Alkanen

Die Erfindung betrifft ein Verfahren zur Herstellung von durch Chloratome und/oder Sulfochloridgruppen substituierten Alkanen durch Umsetzung von Alkanen mit Chlor und gegebenenfalls Schwefeldioxid in einem bestimmten Durchsatzverhältnis und in einem zur Horizontalen geneigten Reaktionsraum, wobei die Ausgangsstoffe im Gleichstrom von unten her durch den Reaktionsraum geleitet werden und der Reaktionsraum in unteren Teil mit Licht der Wellenlängen von 500 bis 700 Nanometern und im oberen Teil mit Licht der Wellenlängen zwischen 200 und 500 Nanometern belichtet wird.

Es ist aus Ullmanns Encyklopädie der technischen Chemie, Band 8, Seiten 353 bis 356, und Band 16, Seiten 562 ff und Lindner, Tenside, Textilhilfsmittel, Waschrohstoffe, 2. Auflage, Band 1, Seiten 708 bis 713, bekannt, Alkane mit mehr als 5 Kohlenstoffatomen (Einzelparaffine) mit Chlor und gegebenenfalls Schwefeldioxid unter Verwendung von thermischer Energie und gegebenenfalls Lichtenergie zu chlorieren und/oder durch Sulfochloridgruppen substituierten Alkanen umzusetzen. Bei solchen Umsetzungen werden in der Regel alle theoretisch möglichen Isomeren gebildet (loc. cit., Band 8, Seite 354, 2. Absatz). Neben den Isomeren desselben Substituierungsgrades, z.B. allen Isomeren des Monochlor-n-pentadecans, werden auch die Isomere der Stufen fortschreitender Substituierung, z.B. Isomere des Dichlor-, Trichlor-, Polychlor-n-pentadecans, gebildet (Überchlorierung bzw. Übersulfochlorierung), obgleich das gesamte Ausgangsparaffin noch nicht umgesetzt ist (Lindner, loc. cit., Seiten 710 bis 713).

In überwiegendem Maße wird in der Technik kein einzelnes Alkan, sondern ein Alkangemisch (Paraffin) in Gestalt von Paraffinwaschen, Crackungsrückständen, ölhaltigen Gemischen fester Paraffine in Schmierölfraktionen (Paraffingatsch), oder Erdölfraktionen, z.B. der Siedegrenzen zwischen 250 und 350°C, Kohlenwasserstoffen der Fischer-Tropsch-Synthese, z.B. der Siedepunkte 195 bis 330°C, oder niedermolekularen Polyolefinen chloriert oder sulfochloriert. Bei solchen Umsetzungen entstehen daher, insbesondere bei den bekannten kontinuierlichen Verfahren (Lindner, loc. cit., Seiten 710 bis 713), in großem Maße Verbindungen, die im Hinblick auf das Molverhältnis der Reaktionspartner einen Überschuß an Chloratomen bzw. Sulfochloridgruppen tragen (polysubstituierte Verbindungen), auf der anderen Seite wird ein entsprechender Anteil an Paraffin nicht umgesetzt. Der Anteil an unumgesetztem und polysubstituiertem Paraffin in z.B. kontinuierlichem Verfahren kann bis zu 70 Gewichtsprozent (berechnet als Ausgangsparaffin), bezogen auf das Gesamtausgangsparaffin im Reaktionsgemisch, betragen.

Solche Gemische sind insbesondere durch ihren Anteil an unumgesetztem Paraffin nachteilig. Dieser Anteil fördert die Trübung des flüssigen, substituierten Paraffins, scheidet sich mit der Zeit ab und stört die Verwendung dieser Paraffine bzw. ihrer Folgeprodukte. In geringerem Maße können ebenfalls die polysubstituierten Paraffine die spätere Verwendung stören. Beispielsweise werden weichmachende Wirkung, Wascheffekt und Netzeffekt entsprechender Textilhilfsmittel, die Schmierwirkung synthetischer Schmieröle oder die emulgierende Wirkung entsprechender Emulgatoren auf Basis solcher Chlorparaffine auf diese Weise verringert und mit solchen Hilfsmitteln behandelte Textilwaren beeinträchtigt. Entsprechend wird die Verwendung solcher Produkte als Lederfettungsmittel beeinträchtigt. Sofern man ein Isomerengemisch bestimmten Substitutionsgrades benötigt, z.B. Monochloralkangemische, so ist die Abtrennung, z.B. durch Destillation, umso schwieriger, je höher der Anteil der übrigen Komponenten des Reaktionsgemisches liegt.

Verfahrensvarianten, durch Temperatursteuerung oder kürzere Reaktionszeit homogen substituierte Paraffine herzustellen, sind in Ausbeute an homogenem Endstoff unbefriedigend, andererseits können je nach Verfahren zusätzliche Schwierigkeiten, z.B. Dechlorierung, Dehydrochlorierung, Crackung, Verkohlung, explosionsartige Reaktion oder Kohlenstoffabscheidungen, auftreten (Ullmann, loc. cit., Band 8, Seiten 354 bis 355; Band 5, Seiten 438 ff).

Ein verbessertes Verfahren in Gestalt einer stufenweisen Führung der Umsetzung in mehreren reaktionsgefäßen ist schon aus Gründen eines einfachen Betriebs und der Wirtschaftlichkeit unbefriedigend. Die Chlorierung mit einem Überschuß an Paraffin, bezogen auf Chlor, (Anchlorierung) ergibt zwar einen geringeren Anteil an polysubstituierten Verbindungen, statt dessen aber höhere Anteile an unumgesetztem Paraffin, das die Weiterverarbeitung des Endgemisches aus vorgenannten Gründen noch weit stärker behindert.

Gegenstand der DAS 22 17 530 ist ein Verfahren zur Herstellung von durch Chloratome und/oder Sulfochloridgruppen substituierten Alkanen mit mehr als 5 Kohlenstoffatomen und mit einer dem Molverhältnis der Ausgangsstoffe entsprechenden Zahl der Substituenten am Alkanmolekül durch Umsetzung von Alkanen mit Chlor oder mit Chlor und Schwefeldioxid, wobei man die Alkane mit einem Durchsatz von 0,1 bis 30 Kilogramm und Chlor oder Chlor und Schwefeldioxid mit einem Durchsatz von 0,1 bis 20 Kilogramm pro Stunde und Liter Reaktionsraum und mit einer Geschwindigkeit der Gasschicht von 2 bis 30 Meter pro Sekunde und einer Verweilzeit der Gasschicht im Reaktionsraum von 2 bis 60 Sekunden von unten nach oben durch einen Reaktionsraum, dessen Längsachse mit der Horizontalen einen Winkel von 1,5° bis 70° bildet, leitet.

Wenn man im Gegensatz zu dieser Verfahrensweise die Reaktionsteilnehmer von oben nach unten durch ein Reaktionsrohr schickt, ist die Durchmischung der Flüssigkeit geringer, die gasförmigen

Reaktionsteilnehmer reagieren ungleichmäßig mit der Flüssigkeit. Führt man die Reaktionsteilnehmer von unten nach oben durch ein senkrecht angeordnetes Rohr, so erzielt man zwar eine gute Durchmischung, jedoch ist für die Aufwärtsbewegung der Flüssigkeit eine sehr hohe Gasgeschwindigkeit erforderlich. Dementsprechend ist, wie die deutsche Auslegeschrift 22 17 530 lehrt, da eine Mindestverweilzeit der Reaktionsgase in dem Rohr für ihren vollständigen Umsatz eingehalten werden muß, eine sehr große Rohrlänge erforderlich. Verwendet man zur Aufteilung der Flüssigkeit Füllkörper in dem Reaktionsrohr, so erzielt man zwar auf kurzer Strecke höhere Umsätze, jedoch ist es bei der Durchführung der Chlorierung bzw. Sulfochlorierung im technischen Maßstab nicht möglich, wie die deutsche Auslegeschrift 22 17 530 lehrt, die auf verhältnismäßig kleinem Raum auftretende Reaktionswärme so abzuführen, daß eine gleichmäßige Temperatur in der Reaktionsflüssigkeit eingehalten wird und die vorgenannten Schwierigkeiten vermieden werden. Es wird ebenfalls angegeben, daß die Reaktion unter Belichtung durchgeführt werden kann, wobei für die Erzeugung des Lichtes vorzugsweise alle im sichtbaren Bereich strahlenden Lichtquellen verwendet werden können. Bevorzugt sind Lichtquellen mit einem hohen Strahlungsanteil im Bereich der Wellenlängen von 300 bis 500 Nanometern. Die Beispiele zeigen, daß jedes Rohrstück des wendelförmigen Reaktionsraumes mit demselben Licht desselben Wellenlängenbereiches belichtet wird. Im Vergleich zu den vorgenannten Verfahren liefert dieses Verfahren auf einfacherem und wirtschaftlicherem Wege das Endstoffgemisch in besserer Raum-Zeit-Ausbeute.

Eine ähnliche Umsetzung beschreibt die deutsche Offenlegungsschrift 24 59 159, wobei ein Teil des den Reaktionsraum verlassenden Reaktionsproduktes in den Reaktor zurückgeführt wird. Auch bei diesem Verfahren wird gegebenenfalls der gesamt Reaktionsraum mit demselben Licht desselben Wellenlängenbereichs belichtet.

Es wurde nun gefunden, daß sich das Verfahren der DAS 22 17 530 weiter ausgestalten läßt, wenn man den ersten Teil des Reaktionsraumes, der 10 bis 50 Prozent der Gesamtlänge der Längsachse des Reaktionsraumes umfaßt, mit Licht, dessen Wellenlängen zwischen 500 und 700 Nanometern liegen, belichtet und dann den restlichen Teil des Reaktionsraumes mit Licht, dessen Wellenlängen überwiegend zwischen 200 und 500 Nanometern liegen, belichtet.

Das Verfahren kann vorteilhaft so durchgeführt werden, daß man eine aus mehreren, zur Horizontalen geneigten Reaktionsrohen, die durch röhrenförmige Krümmer miteinander verbunden sind, bestehende Rohrwendel mit einem Verhältnis der Länge zum Durchmesser von 100 bis 10 000 zu 1 verwendet.

Das Verfahren kann weiterhin vorteilhaft so durchgeführt werden, daß die Längsachse des gesamten Reaktionsraumes oder die einzelnen Reaktionsrohre und gewünschtenfalls die Krümmer mit der Horizontalen einen Winkel von 1,5 bis 30° bilden.

Das Verfahren kann weiterhin vorteilhaft so durchgeführt werden, daß ein Teil des den Reaktionsraum verlassenden Reaktionsgemisches der Reaktion wieder zugeführt wird.

Die umsetzung kann für den Fall der Verwendung von Tetradecan durch die folgenden Formeln wiedergegeben werden:

$$CH_3—[CH_2]_{12}—CH_3 + Cl_2 \longrightarrow C_{14}H_{29}Cl \text{ (Isomerengemisch)} + HCl$$

$$CH_3—[CH_2]_{12}—CH_3 + Cl_2 + SO_2 \longrightarrow C_{14}H_{29}SO_2Cl \text{ (Isomerengemisch)} + HCl$$

$$CH_3—[CH_2]_{12}—CH_3 + 2Cl_2 + SO_2 \longrightarrow C_{14}H_{28}\begin{matrix}—Cl \\ —SO_2Cl\end{matrix} \text{ (Isomerengemisch)} + 2\ HCl$$

Im Vergleich zu den bekannten Verfahren liefert das Verfahren nach der Erfindung überraschend auf einfacherem und wirtschaftlicherem Wege chlorierte und/oder sulfochlorierte Alkane mit einer dem Molverhältnis der Ausgangsstoffe entsprechenden Zahl der Substituenten in besserer Raum-Zeit-Ausbeute. Der Anteil an unumgesetzten Alkanen und an überchlorierten und/oder übersulfochlorierten Verbindungen ist auch im Vergleich zu den Verfahren von DAS 22 17 530 und DOS 24 59 159 geringer. Verfärbungen sind nicht zu beobachten. Alle vorgenannten Schwierigkeiten der Weiterverarbeitung und der Verwendung der Endstoffe oder Folgeprodukte und der damit behandelten Waren sind im Hinblick auf die älteren Verfahren wesentlich verringert. Vorteile des erfindungsgemäßen Verfahrens sind ebenfalls eine gleichmäßigere Reaktionstemperatur und wesentlich kürzere Reaktionszeiten. Ferner ergibt sich ein guter Wärmeübergang von dem Reaktionsgemisch auf die Rohrwandung, was für den einfachen und wirtschaftlichen Abtransport der Reaktionswärme, insbesondere bei großen Durchsätzen, von Bedeutung ist. Alle diese vorteilhaften Eigenschaften sind gerade auch im Hinblick auf die deutsche Auslegeschrift 22 17 530 und die deutsche Offenlegungsschrift 24 59 159 überraschend, denn man hätte erwartet, daß bei Belichtung mit demselben Licht desselben Wellenlängenbereichs, insbesondere bei hohen Durchsätzen, die Reaktionswärme schwieriger durch Kühlung vollständig abgeführt werden kann, so daß Überhitzung des Reaktionsproduktes eintritt bzw. die Umsetzung unvollständig verläuft. Außerdem vermeidet das erfindungsgemäße Verfahren überraschend von Angang an Störungen in der Verteilung der Reaktionskomponenten. Teilchen, deren Stoffaustausch mit der Gasphase — verglichen mit der Masse des reaktionsgemisches — begünstigt ist,

werden in geringerem Maße überchloriert. Solche Teilchen, in Gestalt von Tröpfchen und Schaumlamellen, bilden sich bei der starken Strömung überall im Reaktor. Das erfindungsgemäße Verfahren verhindert so überraschend eine Abscheidung unslöslicher Partikel aus dem homogenen Endgemisch.

Auch in der Anlage selbst stören solche überchlorierten Teilchen. Es ist nämlich kaum zu vermeiden, daß geringe Mengen des Reaktionsgemisches den Reaktionsraum als Aerosol oder Nebel verlassen. Sind darin überchlorierte, bei Raumtemperatur feste Produkte enthalten, so führen sie bei der Aufarbeitung des Abgass zu erheblichen Störungen, da sie die Rohrleitungen zusetzen. Die schlecht löslichen, hochchlorierten Verbindungen sind nur sehr schwer zu entfernen. Auch wird in vorgenanntem Falle bei Sulfochlorierungen die Chlorierung der Alkane in unerünschter Weise begünstigt. Das erfindungsgemäße Verfahren zeigt in diesen Fällen den überraschenden Vorteil, daß trotz hoher Chlorkonzentration in der Gasphase die Reaktion gemäßigter verläuft, keine Kühlprobleme auftreten, die Chlorierung des Alkans bei der Sulfochlorierung nicht über das übliche Maß hinaus begünstigt und der Durchsatz wesentlich erhöht wird, ohne daß Chlor im Abgas enthalten ist. Das erfindungsgemäße Verfahren ist vergleichsweise umweltfreundlicher.

Als Ausgangsalkane verwendet man bevorzugt solche mit 10 bis 30, zweckmäßig 14 bis 24, insbesondere 19 bis 23 Kohlenstoffatomen. Sie können verzweigt oder geradkettig sein und kommen einzeln oder vorteilhaft als Alkangemisch in Betracht. Es kommen beispielsweise als Alkane n-Dodecan, n-Tridecan, n-Tetradecan, n-Pentadecan, n-Hexadecan, n-Heptadecan, n-Octadecan, n-Nonadecan, n-Eicosan, 2,2,3,3-Tetramethylbutan, 2,2,4-Trimethylpentan, n-Tricontan in Frage. Schon aus Wirtschaftlichkeitsgründen wird man im allgemeinen die im Stand der Technik aufgeführten Paraffine verwenden, wobei die Kohlenstoffzahl dann von dem vorgegebenen Paraffin abhängt; so haben die für die Sulfochlorierung verwendeten Erdölfraktionen bevorzugt Gemische von Alkanen unterschiedlicher Kohlenstoffzahl und Isomerie mit einer Durchschnittszahl von 14 bis 18 Kohlenstoffatomen je Molekül.

Die Ausgangsstoffe können in stöchiometrischer Menge verwendet werden, z.B. zur Herstellung von Trichloralkanmonosulfochloriden 4 Mol Chlor und 1 Mol Schwefeldioxid je Mol Alkan. Bei Alkangemischen wie den genannten Paraffinen, beziehen sich die stöchiometrischen Mengen auf das Mol Alkan mit der für das jeweilige Paraffin festgestellten Durchschnittszahl an Kohlenstoffatomen. Bevorzugt wird man für die Herstellung von disubstituierten, trisubstituierten und höher substituierten Alkanen und Paraffinen einen Überschuß an Chlor und gegebenenfalls Schwefeldioxid, bezogen auf Alkan bzw. Paraffin, verwenden; es kommen Mengen über der stöchiometrischen Menge bis zu 130 Gewichtsprozent, zweckmäßig von 105 bis 120 Gewichtsprozent Chlor über der stöchiometrischen Menge, und gegebenenfalls bis zu 200 Gewichtsprozent über der stöchiometrischen Menge, zweckmäßig von 110 bis 170 Gewichtsprozent über der stöchiometrischen Menge Schwefeldioxid, bezogen auf 1 Mol Alkan bzw. Paraffin, in Betracht. Im Falle von Alkangemischen bzw. Paraffinen wird der Berechnung das Durchschnittsmolekulargewicht zugrunde gelegt. Bei der Herstellung von monosubstituierten Verbindungen wird vorteilhaft ein Unterschuß an Chlor (Anchlorierung) und gegebenenfalls Schwefeldioxid verwendet, es kommen Mengen unterhalb der stöchiometrischen Menge bis zu 10 Gewichtsprozent (bezogen auf die stöchiometrische Menge), zweckmäßig von 20 bis 50 Gewichtsprozent (bezogen auf die stöchiometrische Menge) Chlor in Frage.

Bei allen Sulfochlorierungen enthalten die Endstoffe bzw. Endgemische neben Sulfochloridsubstituenten praktisch immer einen kleinen Anteil an Chlorsubstituenten. Es entstehen somit neben reinen Alkansulfochloriden noch Chloralkansulfochloride und Chloralkane in einem entsprechenden Isomerengemisch. Will man diesen Anteil an Chlorsubstituenten möglichst niedrig halten, wählt man einen vorgenannten Unterschuß an Chlor und zweckmäßig ein Verhältnis von 0,5 bis 0,9, vorzugsweise 0,6 bis 0,8 Mol Chlor je Mol Schwefeldioxid.

Das Verfahren nach der Erfindung geht von der Beobachtung aus, daß man die umzusetzenden Stoffe in das untere Ende eines zur Horizontalen aufsteigenden Rohres einleiten und dabei die Gasgeschwindigkeit so hoch halten muß, daß das Gas die Flüssigkeit nach oben drückt und die Flüssigkeit in dünner Schicht aufwärts strömt. Es zeigt sich, daß bei dieser Verfahrensweise die Flüssigkeit, die das Bestreben hat, rückwärts zu fließen, durch das über ihr strömende Gas in eine wellenförmige, rollende Aufwärtsbewegung gerät und gut durchmischt wird, ohne daß eine Rückvermischung in schädlichem Ausmaß eintritt. Die sich aufwärts bewegende Flüssigkeit kommt in gleichmäßigen Kontakt mit dem Gas, und es stellt sich in ihr eine gleichmäßige Temperatur ein. Die Flüssigkeit besteht am Anfang aus den flüssigen Alkanen bzw. im Falle fester Alkane der Alkanschmelze und setzt sich beim Durchströmen des Rohres in steigendem Maße zum Endstoff bzw. Endgemisch um. Es handelt sich somit um eine Umsetzung in einem Zweischichtensystem, wobei die Flüssigkeit ohne erhebliche Rückvermischung gut vermischt wird. Gas wie Flüssigkeit bleiben während der gesamten Reaktionsstrecke schichtenförmig, d.h. eine über ganzen Raumquerschnitt reichende, vollständige Vermischung von Gas und Flüssigkeit tritt an keiner Stelle im Reaktionsraum ein.

Die Vermischung von Gas und Flüssigkeit ist eine Oberflächenvermischung und keine turbulente Durchwirbelung beider Schichten.

Bevorzugt verwendet man Durchsätze von 0,1 bis 25, insbesondere von 0,1 bis 20 Kilogramm Alkan und von 0,1 bis 13, insbesondere von 0,1 bis 5 Kilogramm Chlor oder Chlor und Schwefeldioxid pro Stunde und Liter Reaktionsraum und Geschwindigkeiten der Gasschicht von 2 bis 30, insbesondere

von 3 bis 10 Meter pro Sekunde und der Flüssigkeitsschicht von 0,02 bis 3, insbesondere von 0,03 bis 1 Meter Pro Sekunde. Bevorzugt sind Verweilzeiten von 2 bis 60, insbesondere von 2 bis 30 Sekunden für die Gasschicht und von 60 bis 1 200, insbesondere von 180 bis 900 Sekunden für die flüssige Schicht des Reaktionsgemisches.

Die Reaktion wird im allgemeinen bei einer Temperatur von 20 bis 160°C, drucklos oder unter Druck, zweckmäßig mit einem Gasdruck von 1 bis 4, vorzugsweise von 1,2 bis 2,5 bar, diskontinuierlich oder vorzugsweise kontinuierlich durchgeführt. Im Falle von Chlorierungen sind Reaktionstemperaturen von 60 bis 160°C, im Falle von Sulfochlorierungen von 20 bis 40°C und im Falle von gleichzeitiger Chlorierung und Sulfochlorierung von 25 bis 120°C, insbesondere von 30 bis 90°C, bevorzugt. Gegebenenfalls kann man unter den Reaktionsbedingungen inerte, organische Lösungsmittel wie Chlorkohlenwasserstoffe, z.B. Tetrachlorkohlenstoff, Tetrachloräthylen, Tetrachloräthan und entsprechende Gemische verwenden; es kommen Mengen von 5 bis 70 Gewichtsprozent Lösungsmittel, bezogen auf Alkan bzw. Paraffin, in Betracht.

Zweckmäßig wählt man als Reaktionsraum Rohre mit 10 bis 100, vorzugsweise 20 bis 90 Meter Länge und 0,01 bis 0,10, vorzugsweise 0,015 bis 0,08 Meter Durchmesser (lichte Weite). Der Reaktionsraum hat einen Winkel von 1,5 bis 70°, vorzugsweise von 1,5 bis 30° zwischen seiner Längsachse und der Horizontalen. Vorteilhaft wird der Reaktionsraum aus Rohren in Form einer Spirale gebildet. Ebenfalls kann der Gesamtreaktionsraum aus verschiedenen, hintereinander geschalteten Reaktionsräumen, zweckmäßig in Gestalt von Rohren, bestehen, wobei alle oder ein Teil der Längsachsen der einzelnen Räume (Rohre) jeweils untereinander einen unterschiedlichen, erfindungsgemäßen Winkel mit der Horizontalen bilden. Ein geringerer Teil der Räume (Rohre), im allgemeinen 0 bis 20 Prozent der Gesamtrohrlänge, kann auch horizontal oder mit einem Winkel zwischen 0 und 1,5° oder oberhalb 70° zur Horizontalen geneigt sein, im allgemeinen werden aber solche Räume nicht oberhalb 70°C, sondern zweckmäßiger zwischen 0 und 1,5° geneigt oder vorteilhafter horizontal sein; dieser geringere Teil der Räume besteht zweckmäßig nur als Krümmern, die die einzelnen, mit dem erfindungsgemäßen Neigungswinkel zur Horizontalen geneigten Rohre miteinander verbinden. In allen diesen Fällen entspricht die Längsachse des Gesamtraumes der Verbindungslinie zwischen den Mittelpunkten des Eingangs- und Ausgangsquerschnitts des Gesamtreaktionsraumes, vorteilhaft eines aus mit Krümmern verbundenen geraden Rohrstücken in der Art einer Wendeltreppe konstruierten Gesamtrohres. Als Krümmer werden hier gekrümmte Rohrverbindungen verstanden. Jeweils ein Krümmer verbindet den Ausgang eines Rohres mit dem Eingang des nächsten sich anschließenden Rohres, wobei entsprechend alle Rohre einen, zweckmäßig denselben erfindungsgemäßen Neigungswinkel zur Horizontalen besitzen und die Krümmer a) ebenfalls einen, zweckmäßig den Rohren gleichen Neigungswinkel besitzen, oder b) aus konstruktiven Gründen im rechten Winkel zu den Rohren und horizontal liegen bzw. c) vorteilhafter an den Enden des Krümmers dem Neigungswinkel der sich anschließenden Rohre entsprechen und im übrigen, mittleren Teil des Krümmers horizontal liegen (siehe auch Zeichnung). Bevorzugt ist ein rohrförmiger Reaktionsraum in Gestalt einer Wendel, Wendeltreppe bzw. eines diesem Begriff entsprechenden Treppenhauses, wobei die einzelnen, geraden Rohrstücke den einzelnen Treppenstücken und jeder Krümmer dem sie verbindenden Treppenabsatz entsprechen. Somit liegt der Hauptteil, nämlich das Mittelstück eines jeden Krümmers, der die geraden Rohrstücke quer miteinander verbindet, vorteilhaft horizontal oder mit einem Neigungswinkel in der Regel unter 70°, im allgemeinen von 0 bis 2°, stets quer oder waagrecht zur Richtung der Rohrstücke, aber niemals senkrecht zu dieser Richtung bzw. der Neigungswinkel jedes Rohrstückes zur Horizontalen darf mit dem Neigungswinkel des folgenden Rohrstückes zur Horizontalen nicht in einer Ebene liegen. Bei dieser bevorzugten Ausfurungesform der Wendel oder Wendeltreppe ist eine Zahl von 10 bis 200 Einzelrohren und entsprechend von 9 bis 199 Krümmern vorteilhaft; bevorzugt is ein Verhältnis der Länge zum Durchmesser der Wendel (Rohre und Krümmer) von 100 bis 10 000, insbesondere von 200 bis 8 000 zu 1. Zweckmäßig haben die Krümmer denselben Durchmesser (lichte Weite) wie die Reaktionsrohre, alle Krümmer bzw. Krümmermittelteile untereinander und/oder alle Rohrstücke untereinander dieselben Dimensionen und Neigungswinkel.

Man kann die Reaktionswärme durch Wasserkühlung des Rohrsystems von außen, z.B. mit Hilfe von Doppelmantelrohren, abführen. Man kann vorteilhaft auch mittels eines im Inneren des Reaktionsrohres konzentrisch angeordneten Rohres, das mit Kühlmittel durchflossen wird, kühlen. Dieses innere Kühlrohr braucht nicht, wie die Außenkühlrohre, lichtdurchlässig zu sein, kann deshalb aus metallischem Werkstoff bestehen, so daß das Kühlproblem infolge der höheren Wärmedurchgangszahl des metallischen Werkstoffs gegenüber Glas vereinfacht wird. Durch den Einbau eines konzentrischen Rohres in das Reaktionsrohr wird daneben die Durchmischung und Reaktion von Flüssigkeit und Gas begünstigt.

In einer bevorzugten Auführungsform führt man einen Teil, bevorzugt 3 bis 40 Gewichtsprozent, vorzugsweise 5 bis 20 Gewichtsprozent, des den Reaktionsraum verlassenden Reaktionsgemisches der Reaktion wieder zu und chloriert, sulfochloriert bzw. chloriert und sulfochloriert mit dem frischen Kohlenwasserstoff zusammen erneut. Es hat sich gezeigt, daß eine kontinuierliche Rückführung von 5 bis 20 Prozent des Sulfochlorierungsgemisches schon ausreichen, um nach der Verseifung der Endstoffe zu Produkten mit sehr guter Netzwirkung zu kommen. Bezüglich der Rückführung und Umsetzung können vorteilhaft die in der deutschen Offenlegungsschrift 24 59 159 beschriebenen Bedingungen

und Verfahrensmerkmale verwendet werden. Der Netzwert ist die Zeit in sec, die ein Baumwollscheibchen von 30 mm Durchmesser in 200 ml Lösung, die 1 g Netzmittel enthält, zum Absinken braucht. Die Schaumzahl ist <30 sec. Sie wird nach folgender Methode bestimmt: 0,5 g Substanz werden in einem 250 ml-Schüttelzylinder mit Natronlauge von 20° Baumé auf 100 ml aufgefüllt und 20 mal kräftig in vertikaler Richtung geschüttelt. Die Schaumzahl ist die Zeit in sec, nach der der Schaum noch 5 ml beträgt.

Die Reaktion wird unter Belichtung durchgeführt, wobei für die Erzeugung des Lichtes vorzugsweise alle im sichtbaren Bereich strahlenden Lichtquellen verwendet werden können. So kann z.B. mit Sonnenlicht oder künstlichem Licht, z.B. von Quarzbrennern, Quecksilberdampflampen, Tageslichtlampen, Leuchtstoffröhren, belichtet werden.

Gegebenenfalls verwendet man Tauchlampen, die vom Reaktionsgemisch umspült werden. Durch entsprechende Filter werden zweckmäßig aus der Lichtquelle die gewünschten Wellenlängen allein für die Belichtung herausgefiltert. Der gesamte Reaktionsraum (zweckmäßig Rohrwendel) wird belichtet, wobei die in den Reaktionsraum eintretende Gas- und Flüssigkeitsschicht in einem ersten Teil (unterem Teil) des Reaktionsraumes (der Rohrwendel) mit Licht mit einem hohen Strahlungsanteil im Bereich von Wellenlängen von 500 bis 700, vorzugsweise von 530 bis 680 Nanometern und anschließend in dem restlichen zweiten Teil (oberen Teil) des Reaktionsraumes (der Rohrwendel) mit Licht mit einem hohen Strahlungsanteil im Bereich von Wellenlängen zwischen 200 und 500, vorzugsweise von 330 bis 460 Nanometern belichtet. Die Teilung des Gesamtreaktionsraumes wird in der Gesamtlänge vorgenommen und zwar so, daß der erste Teil 10 bis 50, vorzugsweise 20 bis 40 Prozent der Gesamtlänge der Längsachse des Reaktionsraumes und der restliche zweite Teil entsprechend 50 bis 90, vorzugsweise 60 bis 80 Prozent der Gesamtlänge der Längsachse des Reaktionsraumes entspricht. Der Strahlungsanteil des Lichtes kommt durch den Anteil der genannten Wellenlängen an der Gesamtlichtstärke des Lichtes zum Ausdruck. Der Anteil der vorgenannten Wellengereiche beträgt bei der Belichtung 50 bis 100 Prozent der Gesamtlichtstärke, vorzugsweise für den Wellenlängenbereich von 500 bis 700 Nanometern im ersten Teil des Reaktionsraumes 60 bis 100, insbesondere 80 bis 100 Prozent der Gesamtlichtstärke, für den Wellenlängenbereich zwischen 200 und 500 Nanometern im zweiten Teil des Reaktionsraumes 60 bis 100, insbesondere 80 bis 100 Prozent der Gesamtlichtstärke des im Jeweiligen Teil verwendeten Lichtes. Gegebenenfalls können in einem oder beiden Teilen des Reaktionsraumes einige Stellen, die schlecht zu belichten sind, z.B. der Raum in der Krümmern, nicht belichtet werden, wobei solche nicht belichteten Stellen insgesamt 0 bis 25 Prozent der Gesamtlänge der Längsachse des Gesamtreaktionsraumes enthalten können.

Die Reaktion kann wie folgt durchgeführt werden: Die Ausgangsstoffe werden bei der Reaktionstemperatur, dem Reaktionsdruck und den vorgenannten Durchsätzen von unten her durch den zur Horizontalen geneigten Reaktionsraumes geleitet, wobei im ersten und zweiten Teil mit Licht der jeweiligen erfindungsgemäßen Wellenlängen belichtet wird. Vorteilhaft leitet man das aus dem Reaktionsraum austretende, flüssige Reaktionsgemisch zusammen mit den Reaktionsgasen durch einen mit Füllkörpern gefüllten Rieselturm im Gleichstrom von oben nach unten, um gegebenenfalls den letzten Rest an Ausgangsstoff, z.B. Chlor, in den Reaktionsgasen zur Umsetzung zu bringen. Aus dem Reaktionsgemisch wird im Falle von Einzelalkanen der Endstoff in üblicher Weise, z.B. durch fraktionierte Destillation, abgetrennt. In der Mehrzahl der Fälle, gerade im industriellen Betrieb, handelt es sich um Gemische von Ausgangsalkanen und somit von Endstoffen, die durch Destillation gereinigt werden können, meistens aber der Weiterverarbeitung, z.B. der Verseifung, direkt zugeführt werden. Man verseift z.B. das Sulfochlorierungsprodukt mit wäßriger Natronlauge bei 100°C, kühlt ab und trennt vom ausgeschiedenen festen Kochsalz ab. Man erhält dann eine Alkalisulfonatlösung, die nach Zusatz weniger Prozente eines Antischaummittels ausgezeichnete Netzmittel sind, die durch Netz- und Schaumzahlen charakterisiert werden können.

Die Zeichnungen zeigen eine erfindungsgemäße Anordnung von der Seite (A) und eine Wendeletage von oben (B).

Die nach dem Verfahren der Erfindung herstellbaren substituierten Alkane oder Paraffine sind Schädlingsbekämpfungsmittel, Weichmacher, Lösungsmittel und wertvolle Ausgangsstoffe für die Herstellung solcher Stoffe, sowie von Lederfettungsmitteln, Netzmitteln, Waschmitteln, Schmierölen, Kunstharzen, Gleitmitteln. Bezüglich der Verwendung wird auf vorgenannte Veröffentlichungen und Ullmanns Encycklopädie der technischen Chemie, Band 5, Seiten 435, 437, 448, verwiesen.

Die in den folgenden Beispielen aufgeführten Teile bedeuten Gewichtsteile.

Beispiel 1

a) Es wird der folgende Paraffingatsch verwendet:

| Kohlenstoffzahl | Anteil in Gew.%, bezogen auf Gesamtparaffin | | |
|---|---|---|---|
| $C_{12}$—$C_{15}$ | 0,3% | mittlere Kettenlänge $C_{21,2}H_{44,4}$ | |
| $C_{16}$ | 0,2% | mittleres Molgewicht | 299 |
| $C_{17}$ | 1,1% | Schmelzpunkt | +41°C |
| $C_{18}$ | 4,1% | Dichte bei 45° | 0,765 |
| $C_{19}$ | 11,2% | | |
| $C_{20}$ | 16,2% | | |
| $C_{21}$ | 19,2% | | |
| $C_{22}$ | 17,4% | | |
| $C_{23}$ | 14,1% | | |
| $C_{24}$ | 8,9% | | |
| $C_{25}$ | 4,5% | | |
| $C_{26}$ | 2,2% | | |
| $C_{27}$ | 0,6% | | |

120 Teile Paraffingatsch werden in flüssiger Form zusammen mit 94 Teilen gasförmigem Chlor ständlich in das untere Ende eines Röhrensystems folgender Bauart eingeleitet: 32 gerade Glasrohre von je 2 m Länge und 50 mm lichter Weite sind durch 31 Krümmer derselben lichten Weite wendelförmig in der Art einer Wendeltreppe miteinander verbunden, wobei die Neigung eines jeden Rohres nach oben 2°, der Mittelteil jedes Krümmers horizontal verläuft und Anfang und Ende jedes Krümmers von 0 auf die Neigung 2° des anschließenden Rohres übergehen. Insgesamt betragen die Krümmerstücke mit der Neigung 0 bis 1,5 11,3 Prozent der Gesamtrohrlänge. In jedem Rohr befindet sich konzentrisch ein zweites Glasrohr mit dem Außendurchmesser von 30 mm (lichte Weite 24 mm), durch das Kühlwasser geleitet wird. Aus diese Weise entsteht ein Reaktionsraum mit ringförmigem Querschnitt mit einer Ringbreite von 10 mm (lichte Weite zwischen Innen- und Außenrohrmantel). Jedes der unteren 10 Rohre wird von außen durch eine Leuchtstoffröhre mit einer Leistung von 60 Watt und einem Strahlungsanteil von 80 bis 95 Prozent der Gesamtlichtstärke im Bereich der Wellenlängen von 5 000 bis 7 000 Angström, jedes der 22 restlichen oberen Rohre mit einer Leuchtstoffröhre derselben Leistung und einem Strahlungsanteil von 80 bis 95 Prozent der Gesamtlichstärke im Bereich der Wellenlängen von 3 000 bis 5 000 Angström belichtet. Die Krümmer werden nicht belichtet, so daß der nicht belichtete Anteil der Gesamtrohrlänge 22,5 Prozent beträgt. Das gasförmige Gemisch durchströmt das Röhrensystem mit einem Durchsatz von 0,9 Kilogramm Chlor pro Stunde und Liter Reaktionsraum und treibt die Reaktionsflüssigkeit in dünner Schicht aufwärts. Die Verweilzeit des Gases im Röhrensystem beträgt 10,1 Sekunden, die der Flüssigkeit 6 Minuten. Die Gasgeschwindigkeit beträgt 8,32 m/sec. Die Flüssigkeit hat einen Durchsatz von 1,14 Kilogramm Alkan pro Stunde und Liter Reaktionsraum. Das Reaktionsgemisch erwärmt sich während des Durchgangs durch den Röhrenreaktor durch die freiwerdende Reaktionswärme. Die Temperatur wird bei 75°C gehalten. Reaktionsflüssigkeit und Reaktionsgas werden nach dem Verlassen des Röhrensystems in den oberen Teil eines mit Raschigringen gefüllten Rieselturmes von 1 m Länge und 150 mm Durchmesser geleitet. Die Temperatur der Reaktionsflüssigkeit in dem nicht gekühlten Rieselturm wird nicht erhöht. Flüssigkeit und Gas werden in einem unter der Rieselkolonne befindlichen Behälter getrennt. Die Reaktionsflüssigkeit wird anschließend durch Ausblasen mit Luft von gelöstem Chlorwasserstoff befreit.

Man erhält stündlich 166 Teile Chlorparaffin mit einem Chlorgehalt von 28,3 Gewichtsprozent. Die Ausbeute, bezogen auf eingesetzten Paraffingatsch und auf Chlor, ist praktisch quantitativ in Bezug auf im Molverhältnis der Ausgangsstoffe substituiertem Paraffin, das Abgas besteht aus chlorfreiem Chlorwasserstoff. Das Endgemisch ist eine wasserhelle und bei Raumtemperatur homogene ölige Flüssigkeit und bleibt beim Lagern klar und flüssig.

b) (Vergleich)

Führt man die Reaktion in bekannter Weise kontinuierlich nach der in Lindner, Band 1, Seite 713, 2. Absatz, beschriebenen Verfahrensweise durch, so erhält man ein Endgemisch mit demselben Chlorgehalt, aus dem sich beim Stehen 10 Gewichtsprozent nicht umgesetztes Paraffin in fester Form abscheiden. Ein entsprechender Teil an gebildetem Polychlorparaffin bleibt in Lösung.

c) (Vergleich)

Beleuchtet man das Röhrensystem auf seiner ganzen Länge mit Leuchtröhren der Wellenlänge von 500 bis 700 Nanometer, so kann man stündlich nur 80 Teile Paraffingatsch und 64 Teile gasfömiges Chlor durchleiten. Man erhält 110,5 Teile Chlorparaffin mit einem Chlorgehalt von 28,3 Prozent. 1,4 Teile pro Stunde Chlor verbleiben im Abgas. Erhöht man den Durchsatz auf 120 Teile Gatsch mit 94 Teilen Chlor, so verbleiben über 30 Teile Chlor pro Stunde im Abgas und man erhält keinen einheitlichen Endstoff.

Beispiel 2

a) Es wird ein Paraffin folgender Zusammensetzung verwendet:

| | | | |
|---|---|---|---|
| $C_8$ | 0,04% | mittleres Molgewicht | 215,25 |
| $C_9$ | 0,02% | mittlere Kettenlänge | $C_{15,25}$ |
| $C_{10}$ | 0,06% | Kp 247,5—289,0°C | |
| $C_{11}$ | 1,73% | | |
| $C_{12}$ | 4.21% | Dichte bei 20° 0,768 $n_D^{20}=1{,}431$ | |
| $C_{13}$ | 7,63% | | |
| $C_{14}$ | 16,39% | | |
| $C_{15}$ | 25,20% | | |
| $C_{16}$ | 25,86% | | |
| $C_{17}$ | 15,07% | | |
| $C_{18}$ | 2,19% | | |
| $C_{19}$—$C_{25}$ | 1,60% | | |

120 Teile Paraffin werden stündlich analog Beispiel 1 mit 101 Teilen $Cl_2$ und 80,4 Teilen $SO_2$ nach der Summenformel umgesetzt:

$$C_{15}H_{32} + 2{,}5\ Cl_2 + 2{,}2\ SO_2 \longrightarrow$$

$$C_{15}H_{29,5}Cl_{0,6}(SO_2Cl)_{1,9} + 2{,}5\ HCl + 0{,}3\ SO_2$$

Die Kühlung wird durch Kühlen mit Wasser in den konzentrischen Innenrohren in den ersten 21 Rohren auf 65°C, in den restlichen 11 Rohren auf 45°C gehalten. Man erhält stündlich 237 Teile eines chlorierten, sulfochlorierten Paraffins mit einem Gesamt-Chlorgehalt von 21 Prozent, wovon 5 Prozent direkt an C-Atome gebunden sind und der Rest (16 Prozent) über —$SO_2$-Gruppen mit der C-Kette verknüpft sind. Das Abgas ist frei von Chlor. Die Ausbeute an Sulfochlorid ist praktisch quantitativ in Bezug auf im Molverhältnis der Ausgangsstoffe substituiertem Paraffin.

b) (Vergleich)

Belichtet man das Röhrensystem auf seiner ganzen Länge mit Leichtröhren der Wellenlängen von 500 bis 700 Nanometer, so erhält man bei einem stündlichen Durchsatz von 80 Teilen Paraffin, 68 Teilen Chlor und 60 Teilen $SO_2$ nur 158 Teilen Endprodukt mit einem Gesamt-Chlorgehalt von 21 Prozent, wovon 5 Prozent direkt an C-Atome gebunden sind und der Rest (16 Prozent) über —$SO_2$-Gruppen mit der C-Kette verknüpft sind. Stündliche Mengen von 24 Teilen $SO_2$ und 1,8 Teilen Chlor verbleiben im Abgas.

c) (Vergleich)

Belichtet man den Reaktionsraum auf seiner ganzen Länge mit Lampen von Wellenlängen von 200 bis 500 Nanometern, so kommt es zu örtlichen Überchlorierungen an der Oberfläche und kleine Tröpfchen gelangen in den Gasraum. Man beobachtet Ausfällungen in Endgemisch und vereinzelt Verstopfungen in Teilen des Reaktionsraumes und der Aufarbeitungsanlage.

Beispiel 3

a) 92,1 Teile Paraffin der von Beispiel 2 genannten Zusammensetzung werden zusammen mit 47,1 Teilen gasförmigem Chlor und 120 Teilen $SO_2$ stündlich in die in Beispiel 1 beschriebene Apparatur eingeleitet. Nach dem zehnten 2 m-Rohr werden stündlich weitere 31,5 Teile Chlor eingeleitet. Die Temperatur wird durch Kühlung mit Wasser in den Innenrohren bei 45°C gehalten Flüssiges und gasförmiges Reaktionsgemisch werden nach dem Verlassen des Röhrensystems in den oberen Teil eines mit Raschigringen gefüllten Rieselturmes von 1 m Länge und 150 mm Durchmesser geleitet und anschließend in einem unter der Rieselkolonne befindlichen Behälter getrennt. Aus diesem werden stündlich 12 Teile flüssiges Reaktionsgemisch (12 Gewichtsprozent des das Röhrensystem verlassenden Reaktionsgemisches) entnommen und in das untere Ende des Röhrenreaktors zurückgepumpt. Das Reaktionsgemisch wird durch Ausblasen mit Luft von gelöstem Chlorwasserstoff und $SO_2$ befreit. Die Ausbeute, bezogen auf die Ausgangsstoffe, ist praktisch quantitativ in Bezug auf im Molverhältnis der Ausgangsstoffe substituiertem Paraffin. Man erhält stündlich 180 Teile eines chlorierten, sulfochlorierten Paraffins mit einem Gesamtchlorgehalt von 21,9 Prozent und einem Gehalt an verseifbarem Chlor von 14,8 Prozent. Das Abgas ist chlorfrei. Der Endstoff wird mit 130,5 Teilen 50-prozentiger Natronlauge bei 100°C verseift, das Verseifungsgemisch auf 15°C abgekühlt und von ausgefallenem Kochsalz getrennt. Man erhält 240 Teile einer wäßrigen Paraffinsulfonatlösung, die nach Zusatz des Schaummittels eine Schaumzahl von 25 Sekunden und Netzwerte von 10 Sekunden in Natronlauge von 10° Baumé, von 23 Sekunden in Natronlauge und 20° Baumé und 44 Sekunden in Natronlauge von 30° Baumé ergeben.

b) Unterläßt man die stündliche Rückführung von 12 Teilen Sulfochorierungsgemisch zum Reaktoreingang, so sind die Ergebnisse an Endstoff gleich, aber die Netzwerte der Paraffinsulfonatlösung 12 Sekunden in Natronlauge von 10° Baumé, 39 Sekunden in Natronlauge von 20° Baumé und 62 Sekunden in Natronlauge von 30° Baumé.

(c) (Vergleich)

Belichtet man das Röhrensystem auf seiner ganzen Länge mit Leuchtröhren der Wellenlängen von 500 bis 700 Nanometern, so ist die Umsetzung von Paraffin mit Chlor und $SO_2$ unvollständig und im Abgas befinden sich über 20 Teile Chlor pro Stunde.

d) (Vergleich)

Belichtet man das Röhrensystem auf seiner ganzen Länge mit Leuchtröhren der Wellenlänge von 200 bis 500 Nanometern, so kommt es zu denselben Störungen wie in Beispiel 2 c).

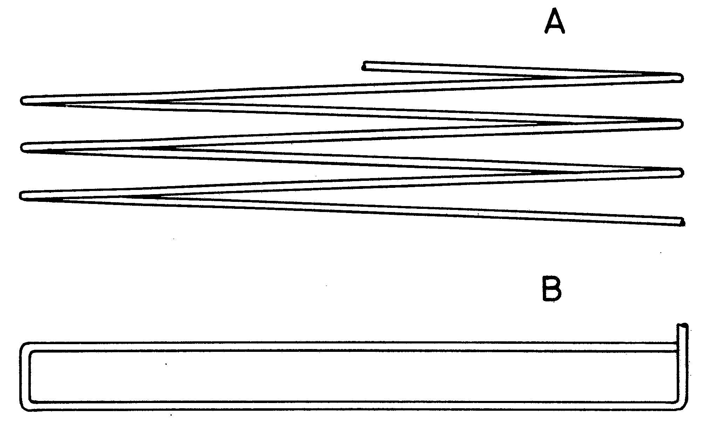

**Patentansprüche**

1. Verfahren zur Herstellung von durch Chloratome und/oder Sulfochloridgruppen substituierten Alkanen mit mehr als 5 Kohlenstoffatomen und mit einer dem Molverhältnis der Ausgangsstoffe entsprechenden Zahl der Substituenten am Alkanmolekül durch Umsetzung von Alkanen mit Chlor oder mit Chlor und Schwefeldioxid, wobei man die Alkane mit einem Durchsatz von 0,1 bis 30 Kilogramm und Chlor oder Chlor und Schwefeldioxid mit einem Durchsatz von 0,1 bis 20 Kilogramm pro Stunde und Liter Reaktionsraum und mit einer Geschwindigkeit der Gasschicht von 2 bis 30 Meter pro Sekunde und einer Verweilzeit der Gasschicht im Reaktionsraum von 2 bis 60 Sekunden von unten nach oben durch einen Reaktionsraum, dessen Längsachse mit der Horizontalen einen Winkel von 1,5 bis 70° bildet, leitet, *dadurch gekennzeichnet*, daß man den ersten Teil des Reaktionsraumes, der 10 bis 50 Prozent der Gesamtlänge der Längsachse des Reaktionsraumes umfaßt, mit Licht, dessen Wellenlängen überwiegend zwischen 500 und 700 Nanometern liegen, belichtet und dann den restlichen Teil des Reaktionsraumes mit Licht, dessen Wellenlängen überwiegend zwischen 200 und 500 Nanometern liegen, belichtet.

2. Verfahren nach Anspruch 1, *dadurch gekennzeichnet*, daß man eine aus mehreren, zur Horizontalen geneigten Reaktionsrohren, die durch röhrenförmige Krümmer miteinander verbunden sind, bestehende Rohrwendel mit einem Verhältnis der Länge zum Durchmesser von 100 bis 10 000 zu 1 verwendet.

3. Verfahren nach Anspruch 1 und 2, *dadurch gekennzeichnet*, daß die Längsachse des Reaktionsraumes oder die Reaktionsrohre und gewünschtenfalls die Krümmer mit der Horizontalen einen Winkel von 1,5 bis 30° bilden.

4. Verfahren nach Anspruch 1, 2 und 3, *dadurch gekennzeichnet*, daß ein Teil des den Reaktionsraum verlassenden Reaktionsgemisches der Reaktion wieder zugeführt wird.

## Claims

1. A process for the preparation of alkanes of more than 5 carbon atoms, which are substituted by chlorine atoms and/or sulfochloride groups, the number of substituents in the alkane molecule corresponding to the molar ratio of the starting materials, by reaction of alkanes with chlorine, or with chlorine and sulfur dioxide, by passing the alkanes, at a throughput of from 0.1 to 30 kilograms per hour per liter of reaction space, and chlorine or chlorine and sulfur dioxide at a throughput of from 0.1 to 20 kilograms per hour per liter of reaction space, upward through a reaction chamber of which the lengthwise axis forms an angle of from 1.5 to 70° with the horizontal, the velocity of the gas layer being from 2 to 30 meters per second and the residence time of the gas layer in the reaction chamber being from 2 to 60 seconds, wherein the first part of the reaction chamber, which accounts for from 10 to 50 per cent of the total length of the lengthwise axis of the reaction chamber, is irradiated with light principally of wavelengths of from 500 to 700 nanometers, and the remaining part of the reaction chamber is irradiated with light principally of wavelengths of from 200 to 500 nanometers.

2. A process as claimed in claim 1, wherein a tubular spiral consisting of several reaction tubes inclined to the horizontal and connected to one another by tubular bends is used, the spiral having a length:diameter ratio of from 100:1 to 10,000:1.

3. A process as claimed in claims 1 and 2, wherein the lengthwise axis of the reaction chamber, or the reaction tubes and, if desired, the bends, forms or form an angle of from 1.5 to 30° with the horizontal.

4. A process as claimed in claims 1, 2 and 3 wherein a part of the reaction mixture leaving the reaction chamber is recycled to the reaction.

## Revendications

1. Procédé pour la préparation d'alcanes substitués par des atomes de chlore et/ou des groupes sulfochlorure, contenant plus de 5 atomes de carbone et ayant un nombre des substituants sur la molécule d'alcane qui correspond au rapport molaire des substances de départ, par réaction d'alcanes avec le chlore ou avec le chlore et l'anhydride sulfureux, en faisant passer de bas en haut, à travers une zone de réaction dont l'axe longitudinal forme avec l'horizontale un angle de 1,5 à 70°, les alcanes avec un débit de 0,1 à 30 kg et le chlore ou le chlore et l'anhydride sulfureux avec un débit de 0,1 à 20 kg par heure et par litre de zone de réaction et avec une vitesse de la couche gazeuse de 2 à 30 m/s et une durée de séjour de la couche gazeuse dans la zone de réaction de 2 à 60 secondes, caractérisé en ce qu'on illumine la première partie de la zone de réaction, qui comprend 10 à 50% de la longueur totale de l'axe longitudinal de la zone de réaction, avec une lumière dont les longueurs d'onde se situent principalement entre 500 et 700 nanomètres, puis on illumine la partie restante de la zone de réaction avec une lumière dont les longueurs d'onde se situent principalement entre 200 et 500 nanomètres.

2. Procédé selon la revendication 1, caractérisé en ce qu'on utilise un serpentin se composant de plusieurs tubes de réaction inclinés par rapport à l'horizontale et reliés entre eux par des raccords tubulaires courbes et présentant un rapport de la longueur au diamètre de 100:1 à 10 000:1.

3. Procédé selon la revendication 1 ou 2, caractérisé en ce que l'axe longitudinal de la zone de réaction ou les tubes de réaction et, si on le désire, les raccords courbes forment avec l'horizontale un angle de 1,5 à 30°.

4. Procédé selon l'une quelconque des revendications 1 à 3, caractérisé en ce qu'une partie du mélange réactionnel qui quitte la zone de réaction est recyclée dans la réaction.

A

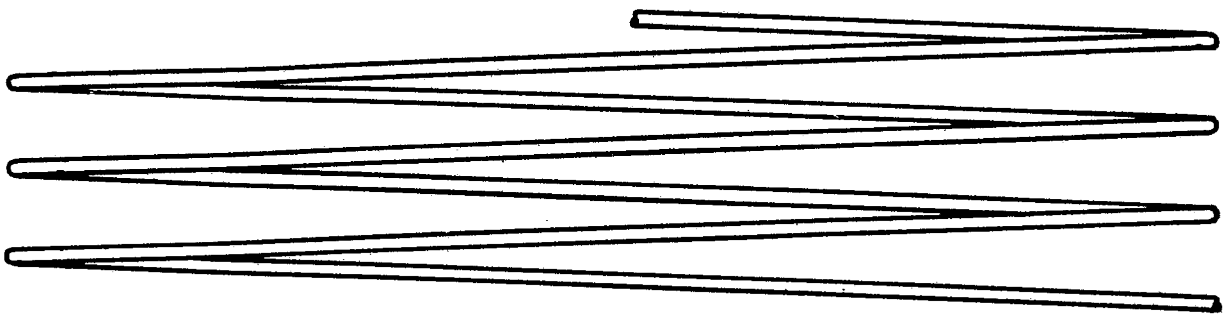

B